(19) Europäisches Patentamt
European Patent Office
Office européen des brevets

(11) **EP 3 508 117 A1**

(12) **EUROPEAN PATENT APPLICATION**

(43) Date of publication:
**10.07.2019 Bulletin 2019/28**

(51) Int Cl.:
*A61B 5/0472* $^{(2006.01)}$    *A61B 5/0456* $^{(2006.01)}$
*A61B 5/024* $^{(2006.01)}$    *A61B 5/02* $^{(2006.01)}$
*A61B 5/00* $^{(2006.01)}$

(21) Application number: **18158139.8**

(22) Date of filing: **22.02.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority: **09.01.2018 US 201862615021 P**

(71) Applicant: **BIOTRONIK SE & Co. KG
12359 Berlin (DE)**

(72) Inventor: **Raven, Joseph Simon
Tigard, OR Oregon 97224 (US)**

(74) Representative: **Keck, Hans-Georg
Biotronik Corporate Services SE
Corporate Intellectual Property
Sieversufer 7 - 9
12359 Berlin (DE)**

(54) **ECG ANALYSIS FOR DIAGNOSIS OF HEART FAILURE AND CARDIOVASCULAR DISEASE USING SIGNALS OBTAINED FROM AN IMPLANTABLE MONITOR**

(57) The invention relates to an implantable device (1) for monitoring of a patient's electrocardiogram (ECG), the device (1) comprising a detection element (2) for determining electrocardiogram signals (S) of a heart (H) of the patient, which signals (S) are indicative of the electrocardiogram (ECG) of the patient, wherein the implantable device (1) comprises a processing unit (3) that is configured to determine from said signals (S) at least one parameter of the electrocardiogram (ECG) or a quantity derived therefrom.

Fig. 1

**Description**

The invention relates to an implantable device for monitoring the heart of a patient.

Worldwide the leading causes of death amongst non-communicable diseases are cardiovascular diseases, many of which contribute to, or are the result of heart failure (HF). The World Health Organization (WHO) Global Health Observatory data repository indicates that nearly 1.8 million deaths will occur as the result of cardiovascular disease (CVD) and this number is projected to climb to 22.2 million by 2030.

US 2004/0116819 A1 discloses an implantable monitoring device which measures and stores the patient's electrocardiogram (ECG) and respiration data via electrodes on the surface of the device.

Particularly, ECG analysis often relies on monitoring by physicians or health care providers or on self-monitoring of the patient to indicate a change in the progression of the patient's disease and the efficacy of their treatment.

In particular, the frequency of ECG data available to physicians on the state of their patient regarding the progression of CVD or HF may be limited to the number of visits and surrounding environmental conditions of the visits made by a patient.

Therefore, it is an objective of the present invention to provide an implantable cardiac monitoring device that helps in generating prognostic indicators of worsening or imminent CVD and HF, as well as providing more accurate diagnosis concerning progression of CVD/HF of the individual patient.

This problem is solved by a device having the features of claim 1. Further embodiments of the present invention are stated in the sub claims and are described below.

According to claim 1 an implantable device for monitoring of a patient's ECG is disclosed, the device comprising a detection element for determining electrocardiogram signals of a heart of the patient when the device is surgically implanted in the (human or animal) patient, wherein signals are indicative of the electrocardiogram of the patient, wherein the implantable device (also denoted as cardiac monitoring device) comprises a processing unit that is configured to determine from said signals at least one parameter of the electrocardiogram or a quantity derived from said at least one parameter/ECG, particularly for: (1) providing prognostic indicators of disease development or early indicators of risk for developing CVD, (2) for diagnosing various cardiovascular diseases or heart failure HF or for determining a progression relating to CVD/HF. The detection element may be a lead.

Thus, utilizing an implantable cardiac monitor, the invention allows assessing various ECG parameters over time, particularly using various algorithms and metrics to generate data which can be made available to the clinician to facilitate the management of patients at risk of, or with, HF or CVD.

The device disclosed herein allows for more information regarding patient disease progression on the basis of more frequent sampling and analysis not normally available to the physician. The types of analyses described herein can efficiently be made by an implantable medical device with the capability of monitoring and recording subcutaneous ECGs thereby providing valuable diagnostic information which has prognostic and diagnostic relevance and provides improved resolution on treatment efficacy.

According to an embodiment, said at least one parameter is a QRS amplitude of the electrocardiogram (ECG).

Particularly, the analysis of the QRS amplitude (i.e. the time interval or duration of the QRS complex) can be utilized as a diagnostic tool for, and an indicator of, Left Ventricular End Diastolic Diameter (LVEDD), Left Ventricular End Systolic Diameter (LVESD), Amylosis, Lower Limb Edema (LLE), and can also be used as an indicator of fibrosis.

Therefore, according to an embodiment of the present invention, the processing unit is configured to record a plurality of successive QRS amplitudes $QRS_{(i-t)}$ during a first sampling period and a plurality of successive QRS amplitudes $QRS_{(i)}$ during a later second sampling period, and to determine a net change of the mean QRS amplitudes $\overline{\Delta QRS}$ between the two sampling periods using the relation $\overline{\Delta QRS} = \overline{QRS}_{(i)} - \overline{QRS}_{(i-t)}$, wherein $\overline{QRS}_{(i)}$ is the average QRS amplitude of the second sampling period, and wherein $\overline{QRS}_{(i-t)}$ is the average QRS amplitude of the earlier first sampling period.

Particularly, according to an embodiment of the present invention, in instances where $\overline{\Delta QRS}$ is negative, the negative value is indicative that factors influencing QRS amplitude like LVEDD (Left Ventricular End Diastolic Diameter), LVESD (Left Ventricular End Systolic Diameter), LVH (left ventricular hypertrophy), LLE (Lower Limb Edema), or Amylosis may develop or worsen and therefore the patient has a poor or worsening prognostic outlook. This uses the assumption that the QRS amplitude will be negatively correlated with the severity of the diseased state.

Furthermore, in an embodiment according to the present invention, the processing unit is configured to attribute a negative net change of the QRS amplitude ($\overline{\Delta QRS}$) to at least one of: LVEDD (Left Ventricular End Diastolic Diameter), LVESD (Left Ventricular End Systolic Diameter), LVH (left ventricular hypertrophy), LLE (Lower Limb Edema), or Amylosis.

Further, according to an embodiment of the present invention, the device is configured to communicate information/data comprising said net change of the mean QRS amplitude ($\overline{\Delta QRS}$), and particularly said attribution, via a telemetry unit of the device to a remote external device (e.g. a device that is used by the patient, a physician or a health care provider to obtain said information data, e.g. for purposes of diagnosis etc.). Such an external device can be a smart phone, a computer, a remote server etc. Here (as well as in the other embodiments), the device may merely provide said information or data and leave attributing this data to a certain CVD/HF to said external device, or medical practitioners operating said external device.

Further, according to an embodiment of the present invention, the processing unit is configured to determine and record a plurality of successive net changes of the mean QRS amplitude $\overline{\Delta QRS}$ as a function of time, particularly from the time at which the cardiac monitoring device has been implanted and started for the first time until the time at which the net change of the mean QRS amplitude $\overline{\Delta QRS}$ has been determined most recently.

Particularly, according to an embodiment of the present invention, the implantable cardiac monitoring device is configured to communicate information / data comprising said plurality of net changes of the QRS amplitude to said external device, particularly for purposes of prognosis/diagnosis. Particularly, the external device may be used to graphically display said net changes for further analysis of the latter. This can be used to provide diagnostic information about the efficacy of treatment and patient prognosis / diagnosis.

Furthermore, R-R variability has longed been recognized as an indicator of the health or tone of the autonomic nervous system and may serve as an indicator in the progression of CVD/HF. Therefore, according to a further embodiment of the present invention, said at least one parameter is an R-R duration of the electrocardiogram (ECG).

Particularly, according to an embodiment the present invention, the processing unit is configured to determine and record a plurality of successive R-R durations $Rd_i$.

Further, according to an embodiment of the present invention, the processing unit is configured to determine a measure $\overline{Rd}$, particularly a mean, of said plurality of R-R durations, wherein the processing unit is further configured to determine a variance $S^2_{(Rd)}$ of said measure using the relation $S^2_{(Rd)} = \frac{1}{n-1}\sum_{i=1}^{n}(Rd_i - \overline{Rd})^2$, wherein n is the number of recorded R-R durations, $Rd_i$ is the i-th recorded R-R duration, and $\overline{Rd}$ is the mean of the plurality of $Rd_i$ samples.

Furthermore, in an embodiment, the processing unit is configured to determine and record a plurality of variances $\left(S^2_{(Rd)}\right)$ for measures of R-R variance across time.

Further, according to an embodiment of the present invention, the processing unit is configured to determine and record at least one or a plurality of F values according to $F = \frac{S^2_{Rd(\alpha)}}{S^2_{Rd(\beta)}}$, wherein $S^2_{Rd(\alpha)}$ is a variance of said plurality of variances that has been determined more recently than the variance $S^2_{Rd(\beta)}$, which is a variance of said plurality of variances, too. Particularly $S^2_{Rd(\beta)}$ is the initial variance determined and recorded first.

In an embodiment, the device is configured to allow for selecting/programming the time between the earlier and the more recent variance used to determine said at least one F value (or said plurality of F values).

Particularly, F values greater than one indicate an increase in R-R variability which may indicate an improvement in patient prognosis, whereas values less than one indicate a negative prognosis. Values of 1 are diagnostic of no observed change.

Thus, in an embodiment according to the present invention, the processing unit is configured to attribute $F$ values greater than one to an improvement in patient prognosis concerning CVD/HF, and to attribute $F$ values less than one to a negative prognosis concerning CVD/HF. Under these conditions a noise floor or assessment of statistical significance may be selectively programmed, non-selectively programmed, or omitted to determine the magnitude of change, above or below zero, to be considered as a clinically relevant measure.

Further, in an embodiment according to the present invention, the device is configured to communicate information data comprising said at least one $F$ value or said plurality of $F$ values, and particularly said attribution, via the telemetry unit of the device to said external device, e.g. for purposes of diagnosis (see also above). Particularly, the $F$ value(s) may be graphically displayed using said external device, e.g. for the purpose of diagnoses etc. Further, according to an embodiment of the present invention, the processing unit is configured to determine and record a difference $\Delta F$ between two of the recorded $F$ values $Fx$ and $Fy$ according to $\Delta F = Fy - Fx$, wherein $Fx$ has been determined and recorded before $Fy$, and wherein particularly the processing unit is configured to determine and record a plurality of such differences of recorded $F$ values.

In an embodiment, the device is configured to allow for selecting and / or programming the time between the *F* value *Fx* and the respective more recent *F* value *Fy*, so that particularly a history of differences *ΔF* can be generated.

Specifically, the difference *ΔF* may provide an index on the progression of autonomic dysfunction over time by comparing two programmable/selectable results from the stored *F* values, e.g. *Fx* and *Fy*. Negative values for *ΔF* can be used as an index of poor prognosis and are diagnostic of a progression of CDV/HF.

Thus, in an embodiment, the processing unit is configured to attribute negative values for ΔF to a progression of CDV/HF. Under these conditions a noise floor or assessment of statistical significance may be selectively programmed, non-selectively programmed, or omitted to determine the magnitude of change, above or below zero, to be considered as a clinically relevant measure.

Further, in an embodiment according to the present invention, the device is configured to communicate information data comprising said at least one difference ΔF or said plurality of differences ΔF, and particularly said attribution, via the telemetry unit of the device to the external device, e.g. for purposes of prognosis / diagnosis (see also above). Particularly, the difference(s) ΔF may be graphically displayed using said external device, e.g. for the purposes of prognoses/ diagnoses etc.

Furthermore, episodes and duration of tachycardia are known to increase as the heart attempts to make up for its mechanical failings; specifically compensatory rate changes to maintain a constant output. Tachycardia may also be tracked utilizing the variable *Rd* as well. However, instead of calculating a variance to the population of samples, a mean will be generated, as shown below.

Therefore, according to an embodiment of the present invention, the processing unit is configured to determine and record a mean ($\overline{Rd_0}$), of said plurality of R-R durations according to $$\overline{Rd}_o = \frac{1}{n}\left(\sum_{i=1}^{n} Rd_i\right),$$ wherein *n* is the number of recorded R-R durations, and $Rd_i$ is the i-th recorded R-R duration.

Particularly, in an embodiment, the device is configured to allow for selecting/programming said number of recorded R-R durations (samples) or the sampling duration. However, said number (sampling duration) may also be fixed.

Further, according to an embodiment of the present invention, the processing unit is configured to determine and record a plurality of means ($\overline{Rd_0}$) of R-R durations as a function of time.

Furthermore, according to an embodiment of the present invention, the processing unit is configured to determine and record a difference ΔRd between two of the recorded means $\overline{Rd_x}$ and $\overline{Rd_y}$ according to ΔRd = $\overline{Rd_y}$ - $\overline{Rd_x}$, wherein $\overline{Rd_x}$ has been determined and recorded before $\overline{Rd_y}$, and wherein the processing unit is configured to determine and record a plurality of such differences ΔRd of recorded means.

In an embodiment, the device is configured to allow for selecting and / or programming the time between the means $\overline{Rd_x}$ and $\overline{Rd_y}$, so that particularly a history of differences *ΔRd* can be generated and recorded.

Particularly, a value of the respective difference ΔRd being smaller than zero or a negative correlation between time and the differences $\Delta Rd_0$ may be diagnostic of a worsening CVD/HF as well as tachycardia alone. Thus, in an embodiment, the processing unit may be configured to attribute negative values for *ΔRd* as well as a negative relationship between time and $\overline{Rd_0}$ to a progression of Tachycardia, CVD, or HF. Under these conditions a noise floor or assessment of statistical significance may be selectively programmed, non-selectively programmed, or omitted to determine the magnitude of change, above or below zero, to be considered as a clinically relevant measure.

Further, in an embodiment, the device is configured to communicate information data comprising said at least one difference *ΔRd* or said plurality of differences *ΔRd,* and particularly said attribution, via the telemetry unit of the device to the external device, e.g. for purposes of prognosis/diagnosis (see also above). Particularly, the difference(s) *ΔRd* may be graphically displayed using said external device, e.g. for the purposes of prognoses/diagnoses etc.

Furthermore, Q-T interval elongation may be seen as an indicator of pump failure, electrical conduction changes, ischaemia, or ventricular aneurysm. Therefore, according to an embodiment of the present invention, said at least one parameter is a Q-T duration.

Furthermore, according to an embodiment of the present invention, the processing unit is configured to determine and record a plurality of successive or non-successive Q-T durations $QT_i$.

Particularly, this may be most easily implemented by measuring from the peak (absolute maximum for each complex) as well as other methods such as a start or end detection method. In the instance the Q wave is not present or has poor resolution the P-wave or R-wave may be utilized as a non-superior alternative. The number of samples or sampling duration may be a fixed or selectable/programmable feature.

Particularly, according to an embodiment of the present invention, the processing unit is configured to determine a mean $\overline{QT_0}$ of said plurality of Q-T durations according to $$\overline{QT}_o = \frac{1}{n}\left(\sum_{i=1}^{n} QT_i\right),$$ wherein *n* is the number of recorded Q-T durations, and $QT_i$ is the i-th recorded Q-T duration.

Particularly, in an embodiment, said number of recorded Q-T duration (samples) or the sampling duration may be fixed or selectable/programmable.

Furthermore, according to an embodiment of the present invention, the processing unit is configured to determine and record a plurality of means $\overline{QT_0}$ of Q-T durations as a function of time.

Further, according to an embodiment, the processing unit is configured to determine and record a difference $\Delta QT$ between two of the recorded means $\overline{QT_x}$ and $\overline{QT_y}$ according to $\Delta QT = \overline{QT_y} - \overline{QT_x}$, wherein $\overline{QT_x}$ has been determined and recorded before $\overline{QT_y}$, wherein particularly the processing unit is configured to determine and record a plurality of differences $\Delta QT$ between [respectively two] recorded means. Preferably, in an embodiment, the device is configured to allow for selecting/programming the time between the means $\overline{QT_x}$ and $\overline{QT_y}$, so that desired selectable history of differences $\Delta QT$ can be generated.

Particularly, a value greater than zero for $\Delta QT$, or a positive correlation between time and the means $\overline{QT_0}$, may be diagnostic of worsening CVD/HF. Under these conditions a noise floor or assessment of statistical significance may be selectively programmed, non-selectively programmed, or omitted to determine the magnitude of change, above or below zero, to be considered as a clinically relevant measure.

Thus, in an embodiment, the processing unit is configured to attribute positive values for $\Delta QT$ or a positive correlation between time and the means $\overline{QT_0}$ to a worsening of one of: pump failure, electrical conduction changes, ischaemia, or ventricular aneurysm.

Further, in an embodiment of the present invention, the device is configured to communicate information data comprising said at least one difference $\Delta QT$ or said plurality of differences $\Delta QT$, and particularly said attribution, via the telemetry unit of the device to the external device, e.g. for purposes of prognosis /diagnosis (see also above). Particularly, the difference(s) $\Delta QT$ may be graphically displayed using said external device, e.g. for the purposes of prognoses / diagnoses etc.

Finally, an R:S wave amplitude ratio may be utilized as an index of left ventricular hypertrophy (LVH) and right ventricular hypertrophy (RVH); which is in many cases a precursor and progressive indicator of CVD and HF. Thus, according to an embodiment of the present invention, said at least one parameter is an R:S ratio ($R{:}S$) defined as the

$$(R{:}S) = \frac{|R_{peak}|}{|S_{peak}|},$$

ratio between the absolute value of the R complex and the S complex, i.e.,

Particularly, according to an embodiment of the present invention, the processing unit is configured to determine and record a plurality of successive R:S ratios (R:S).

Further, according to an embodiment of the present invention, the processing unit is configured to determine

$$\overline{(R{:}S)}_0 = \frac{1}{n}\left(\sum_{i=1}^{n}(R{:}S)_i\right),$$

a mean $\overline{(R{:}S)}_0$ of said plurality of R:S ratios according to wherein n is the number of recorded R:S ratios, and $(R{:}S)_i$ is the i-th recorded R:S ratio.

Particularly, in an embodiment, said number of recorded R:S ratios (samples) or the sampling duration may be fixed or selectable/programmable.

Furthermore, according to an embodiment of the present invention, the processing unit is configured to determine and record a plurality of means $\overline{(R{:}S)}_0$ of R:S ratios as a function of time.

Particularly, in an embodiment, the processing unit is configured to determine a difference $\Delta(R{:}S)$ between two of the recorded means $\overline{(R{:}S)}_x$ and $\overline{(R{:}S)}_y$ according to $\Delta(R{:}S) = \overline{(R{:}S)}_y - \overline{(R{:}S)}_x$, wherein $\overline{(R{:}S)}_x$ has been determined and recorded before $\overline{(R{:}S)}_y$, and wherein particularly the processing unit is configured to determine and record a plurality of such differences $\Delta(R{:}S)$ between recorded means.

Preferably, in an embodiment of the present invention, the device is configured to allow for selecting/programming the time between the means $\overline{(R{:}S)}_x$ and $\overline{(R{:}S)}_y$, so that particularly a history of differences $\Delta(R{:}S)$ can be generated.

In particular, devices implanted with the ECG axis oriented parallel to the midline or in a positive deviation toward the left of the midline but less than perpendicular to midline, will exhibit an increase in the R:S ratio over time in the event RVH is worsening, while the value will decrease from the time of implant if LVH is worsening. The same formulae and methodology associated with comparing values, as performed for Q-T elongation and R-R duration, may be utilized to provide this diagnostic data, i.e., a value greater than zero for the difference $\Delta(R{:}S)$ or a positive correlation between time and the means $\overline{(R{:}S)}_0$ can be diagnostic of worsening of RVH, whereas a value smaller than zero for the difference $\Delta(R{:}S)$ or a negative correlation between time and the means $\overline{(R{:}S)}_0$ can be diagnostic of worsening of LVH. Under these conditions a noise floor or assessment of statistical significance may be selectively programmed, non-selectively programmed, or omitted to determine the magnitude of change, above or below zero, to be considered as a clinically relevant measure.

Thus, in an embodiment, the processing unit is configured to attribute a value greater than zero for the difference

$\Delta(R{:}S)$ or a positive correlation between time and the means $\overline{(R{:}S)}_0$ to a worsening of RVH, and to attribute a value smaller than zero for the difference $\Delta(R{:}S)$ or a negative correlation between time and the means $\overline{(R{:}S)}_0$ to a worsening of LVH.

Further, in an embodiment, the device is configured to communicate information data comprising said at least one difference $\Delta(R{:}S)$ or said plurality of differences $\Delta(R{:}S)$, and particularly said attribution, via the telemetry unit of the device to the external device, e.g. for purposes of prognosis / diagnosis (see also above). Particularly, the difference(s) $\Delta(R{:}S)$ may be graphically displayed using said external device, e.g. for the purposes of prognoses / diagnoses etc.

Particularly, the device according to the invention can be configured to determine and record an arbitrary combination of the above-discussed parameters/quantities, particularly depending on the specific needs of the individual patient.

Furthermore, detailed embodiments and features of the present invention will be described below with reference to the Figures, wherein

Fig. 1    shows a schematic representation of a device according to the invention as well as an electrocardiogram detected by the device;

Fig. 2    shows an algorithm for determining a difference (net change) between average QRS amplitudes carried out by a processing unit of the device according to the invention;

Fig. 3    shows an algorithm for determining a variance of a measure for an R-R duration carried out by a processing unit of the device according to the invention;

Fig. 4    shows an algorithm for conducting an *F* test and for determining a difference between *F* values;

Fig. 5    shows an algorithm for determining a difference (net change) between average R-R durations amplitudes carried out by a processing unit of the device accord;

Fig. 6    shows an algorithm for determining a difference (net change) between average Q-T durations carried out by a processing unit of the device according to the invention; and

Fig. 7    shows an algorithm for determining a difference (net change) between average R:S ratios carried out by a processing unit of the device according to the invention.

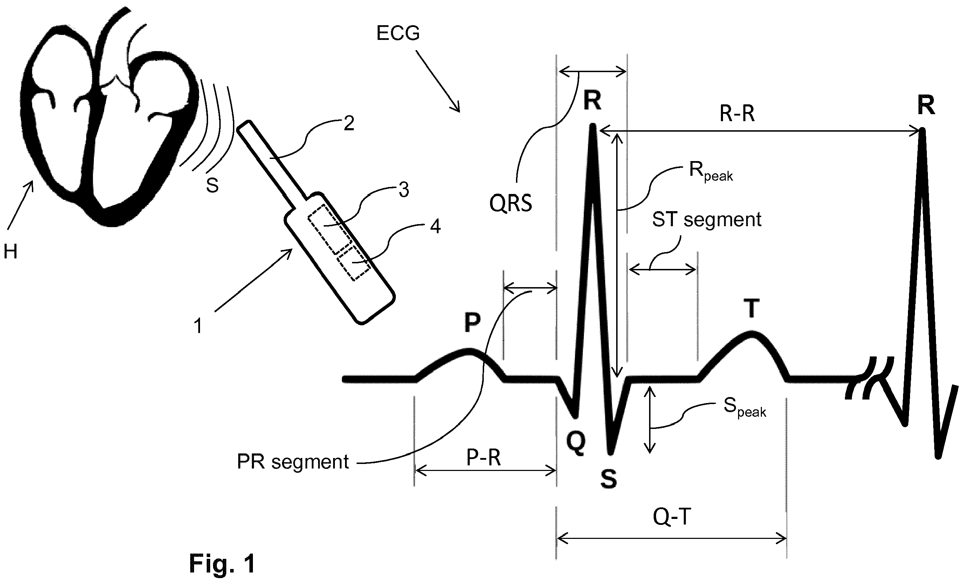

Fig. 1 shows an embodiment of an implantable device 1 for monitoring of a patient's electrocardiogram (ECG) according to the present invention.

The device 1 comprises a detection element (e.g. a lead or a comparable suitable means) 2 for determining electrocardiogram signals S of a heart H of a human or animal patient when the device is implanted in the vicinity of said heart H. The device 1 thus forms a subcutaneous implantable device 1. The signals S are indicative of the electrocardiogram ECG of the patient, which ECG is indicated on the right hand side of Fig. 1. The ECG shows the P wave, QRS complex, the T wave, and a further succeeding QRS complex (the P wave is omitted here) in order to indicate an R-R interval. The U wave following the T wave is not shown. Also indicated is the P-R interval, the QRS amplitude (the sum of the $R_{peak}$ and $S_{peak}$ as indicated), QRS duration, and the Q-T inverval.

Furthermore, the cardiac monitoring device 1 comprises a processing unit 3 that is configured to determine from said signals S at least one parameter of the electrocardiogram ECG or a quantity derived from such a parameter, the ECG, particularly for prognosis or diagnosing of CVD or HF or for determining a progress in HF/CVD.

Particularly, the processing unit 3 is configured to conduct algorithms for determining said parameters or quantities, particularly for monitoring CVD/HF as well be described in more detail below.

Further, particularly, the device 1 comprises a telemetry unit 4, via which said parameter and/or quantities generated by the processing unit 3 can be transmitted to a remote external device located outside the human or animal body (e.g. a smart phone, a computer, or a remote server), via which said parameters/quantities can be graphically displayed and/or further analyzed in order to prognose/diagnose and evaluate progression of CVD, HF, or other pathologies referenced previously, of the patient.

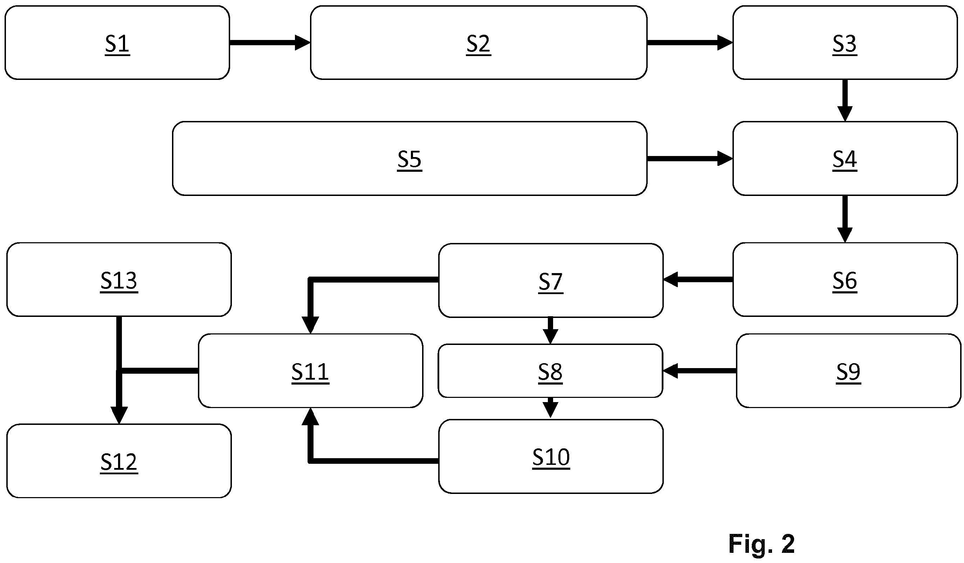

Fig. 2 illustrates an example method for establishing the algorithm to determine a difference (net change) between average QRS amplitudes carried out by a processing unit 3. Here, the electrocardiogram signal is detected by said detection element 2 (step S1). The processing unit 3 detects the QRS complexes (QRS Amplitude Detection Algorithm, step S2) in the received ECG signals (S1) and forms corresponding output variables (QRS output variable, step S3) which are recorded (buffered outputs, step S4). The processing unit 3 further allows an operator to program/select (step S5) the sampling period (programmable sampling period (buffer size)), i.e., the number n of samples, or the period of time over-which samples, are averaged to determine (average calculation, step S6), an average QRS amplitude $\overline{QRS}_{(i)}$ (step S7). The determined averages are recorded (buffered outputs, step S8), wherein in turn the number of recorded averages can be programmed/selected (programmable buffer size, step S9). From these recorded averages an earlier average is selected (prior output value $\overline{QRS}_{(i-t_n)}$, step S10) and a net change (difference) of the QRS amplitude $\varDelta QRS$ is determined (final output, step S11) according to

$$\text{Eq (1):} \qquad\qquad \overline{\varDelta QRS} = \overline{QRS}_{(i)} - \overline{QRS}_{(i-t)}$$

wherein (i) indicates the more recent sampling period over which QRS amplitudes have been collected and averaged, while (i-t) indicates the prior sampling period which may be fixed or programmed. The determined net changes $\varDelta QRS$ in QRS amplitude over the time between the considered sampling periods are recorded/stored (stored history, step S12) to have a history of these values for later analysis, wherein the number of stored net changes $\varDelta QRS$ can be programmed (programmable buffer size, step S13).

Equation (1) provides the means by which QRS amplitudes may be evaluated as an indicator of CVD/HF. Particularly, in instances where $\varDelta \overline{QRS}$ is negative, the negative value may suggest that factors influencing QRS amplitude like LVEDD, LVESD, LVH, LLE, or Amylosis may developing or worsening and therefore the patient has a poor or worsening prognostic outlook. This makes an educated assumption that QRS amplitude will be negatively correlated with the severity of the diseased state.

Additionally, by plotting or tracking the $\overline{QRS}$ or $\varDelta \overline{QRS}$ measurements over time (from implant to the most recent sample) one may be able to provide diagnostic information about the efficacy of treatment and patient prognosis.

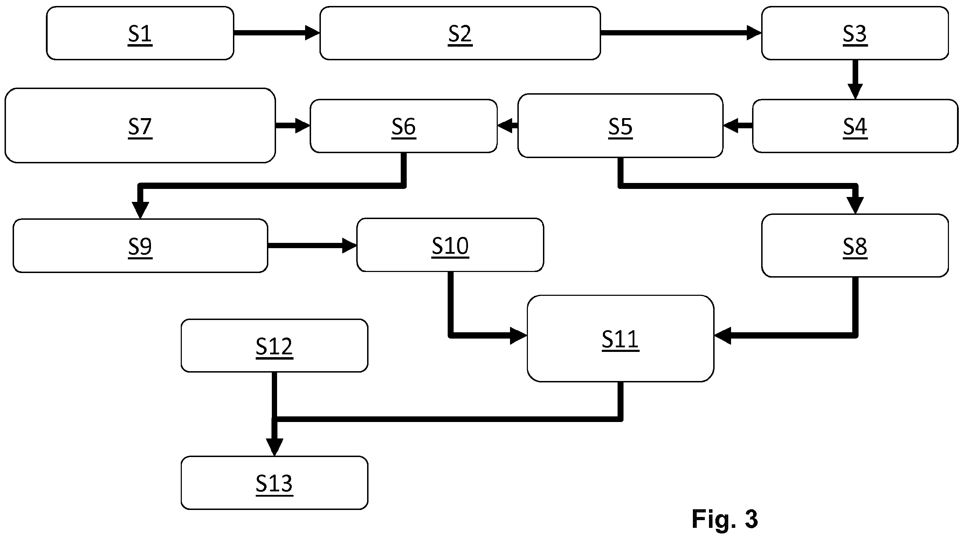

Furthermore, Equations (2-1), (2-2), and (2-3) stated below provide the means by which R-R variability in the detected ECG (cf. Fig. 1) may be evaluated as an indicator of CVD/HF. This is used in an embodiment of the present invention according to Fig. 3, wherein the processing unit 3 is configured to conduct an algorithm for calculating a variance $S^2_{(Rd)}$ of a measure $\overline{Rd}$ for the R-R interval (duration) as indicated in Fig. 1.

For this, according to Fig. 3, the electrocardiogram signal S is detected by said detection element 2 (step S1), and the processing unit 3 detects the QRS complexes (QRS detection algorithm, step S2) in the received ECG signals S and uses time stamps for marking two successive R waves (R-wave marker time stamp, step S3), which are recorded (buffered outputs (2-sample), step S4) and used to determine the corresponding R-R interval (delta of outputs calculation (R-R interval), step S5), which is recorded (buffered delta outputs, step S6), wherein the number of recorded R-R intervals can be selected/programmed (programmable sampling period (buffer size), step S7).

The recorded R-R intervals (S6) are averaged (average calculation of deltas, step S9) to form a measure (e.g. average) $\overline{Rd}$ of the R-R intervals (output $\overline{Rd}$, step S10) which is used in conjunction with the individual R-R intervals (most recent delta $Rd_i$, step S8) that have been determined in step S5, respectively, in order to determine a variance of the measure $\overline{Rd}$ according to

$$\text{Eq (2-1):} \qquad\qquad S^2_{(Rd)} = \frac{1}{n-1}\sum_{i=1}^{n}(Rd_i - \overline{Rd})^2$$

wherein n denotes the number of samples.

The resultant variance $S^2_{(Rd)}$ from Eq (2-1) is then recorded (stored history, step S13) for further analysis and tracking, wherein the number of stored variances $S^2_{(Rd)}$ can be selected/programmed (programmable buffer size, step S12).

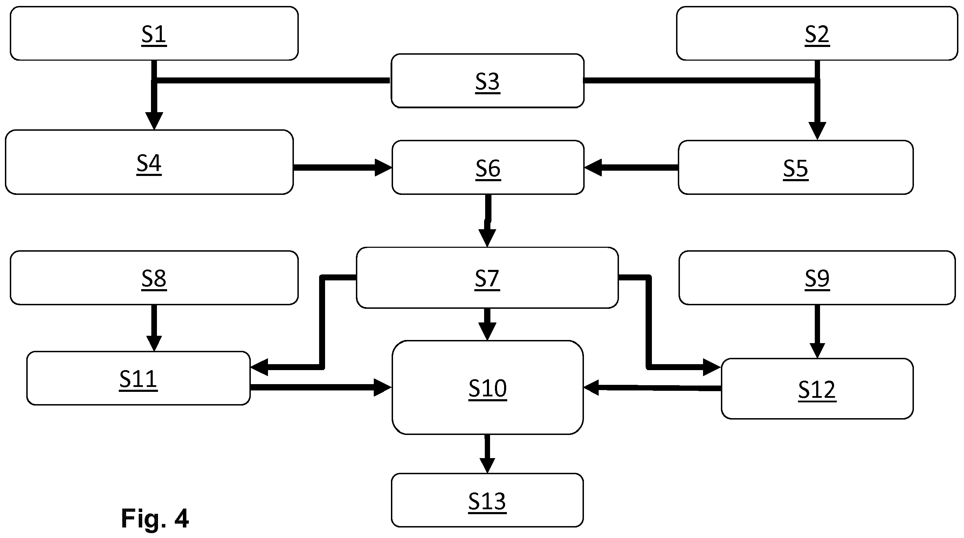

Further, according to an embodiment shown in Fig. 4, stored values (stored history $S^2_{Rd}$, S3) for the variance may then be compared to one another using an F-Test which may indicate the relative change in R-R variability across time. Here, more recent variance values $S^2_{Rd(\alpha)}$ are used (S4) together with an earlier (or the earliest measured) value

$$S^2_{Rd(\beta)}$$ (S5) in order to determine corresponding F values (F-test calculation, step S6) according to

$$\text{Eq (2-2):} \qquad F = \frac{S^2_{Rd(\alpha)}}{S^2_{Rd(\beta)}}$$

F values greater than one indicate an increase in R-R variability which may indicate an improvement in patient prognosis, whereas values less than one indicate a negative prognosis. Values of 1 are diagnostic of no observed change.

The time between each compared measure in Eq. (2-2) is particularly programmable/selectable (programmable sample period; S1, S2). Multiple comparisons may also be made and displayed for additional diagnostic and statistical data.

Additionally, as indicated in Fig. 4, the products of Eq. (2-2) may be stored (stored history, S7) and used for further analysis and tracking. Specifically, Equation (2-3) may provide an index on the progression of autonomic dysfunction over time by comparing two programmable/selectable results (programmable sample period; S8, S9, programmed recent value *Fy,* S11, and programmed historic value, S12) from the stored products (S7) from Eq (2-2), i.e., by calculating (change calculation *ΔF*, step S10)

$$\text{Eq (2-3):} \qquad \Delta F = Fy - Fx.$$

The time between two programmable/selectable results is, according to embodiments of the invention, a period of time that has elapsed between the *Fx* and *Fy* measures. It could also be a time interval in that the device is programmed to assess the difference between *x* and *y.* For example, a programmed interval of three months means that the device is programmed to compare *x* to *y* at three month intervals. Alternatively, the device may be programmed such that it continuously compares each progressive measure to a value that occurred three months earlier.

Here, particularly, negative values for *ΔF* may be used as an index of poor prognosis and are diagnostic of a progression of CDV/HF. The time between each compared measure in Eq (2-3) may be programmable/selectable. Multiple comparisons may also be made and displayed for additional diagnostic and statistical data. The values may be stored (stored history, S13).

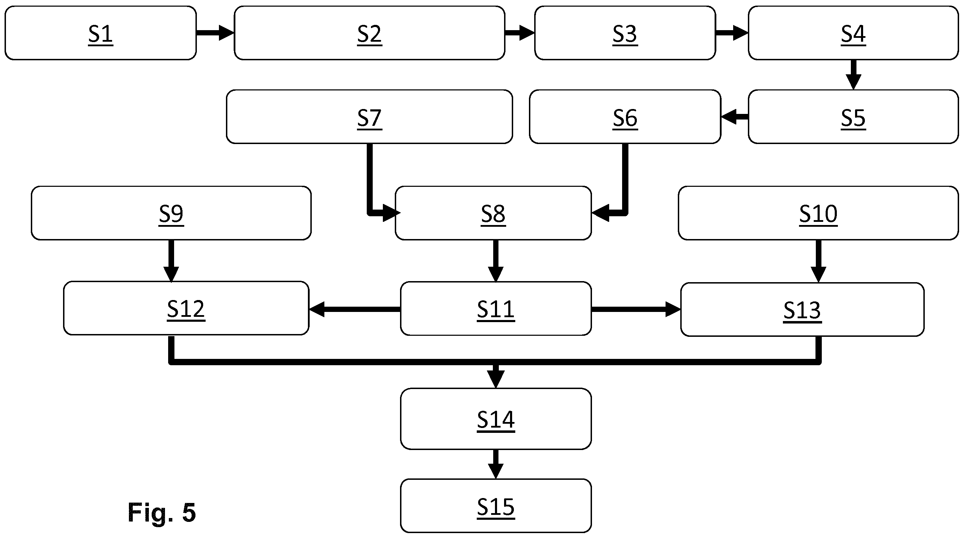

Furthermore, according to an embodiment shown in Fig. 5, tachycardia may also be tracked utilizing the variable *Rd* as well. However, instead of calculating a variance to the population of samples a mean $\overline{Rd}_o$ will be generated, as shown in Equation (3-1).

Also here, as indicated in Fig. 5, the electrocardiogram signal S is detected by said detection element 2 (SI), and the processing unit 3 detects the QRS complexes (QRS detection algorithm, S2) in the received ECG signals S and uses time stamps for marking two successive R waves (QRS marker time stamp, S3), which are recorded (buffered outputs (2-sample), S4) and used to determine the corresponding R-R interval (delta of outputs, S5), which is recorded (buffered delta outputs, S6), wherein the number of recorded R-R intervals can be selected/programmed (programmable sample period, S7).

The recorded R-R intervals $Rd_i$ (step S6) are averaged ($\overline{Rd}_o$ calculation, S8) to form a mean $\overline{Rd}_o$ of the R-R intervals according to

$$\text{Eq (3-1):} \qquad \overline{Rd}_o = \frac{1}{n}\left(\sum_{i=1}^{n} Rd_i\right)$$

wherein the number n of samples or the sampling duration may be selectable/programmable (S7).

The determined means are recorded ($\overline{Rd}_o$ stored history, S11) to have a history of means and differences *ΔRd* are determined (*ΔRd* calculation, S14) according to

$$\text{Eq (3-2):} \qquad \Delta Rd = \overline{Rd}_y - \overline{Rd}_x.$$

Using programmed values (S12, S13), namely a more recent value $\overline{Rd}_y$ and an earlier value $\overline{Rd}_x$.

Particularly, the individual differences *ΔRd* are recorded (stored history, S15) and may be displayed graphically or as calculated by Equation (3-2).

A value smaller than zero for Eq (3-2) or a negative correlation between time and the values obtained from Eq (3-1) may be diagnostic of worsening CVD/HF.

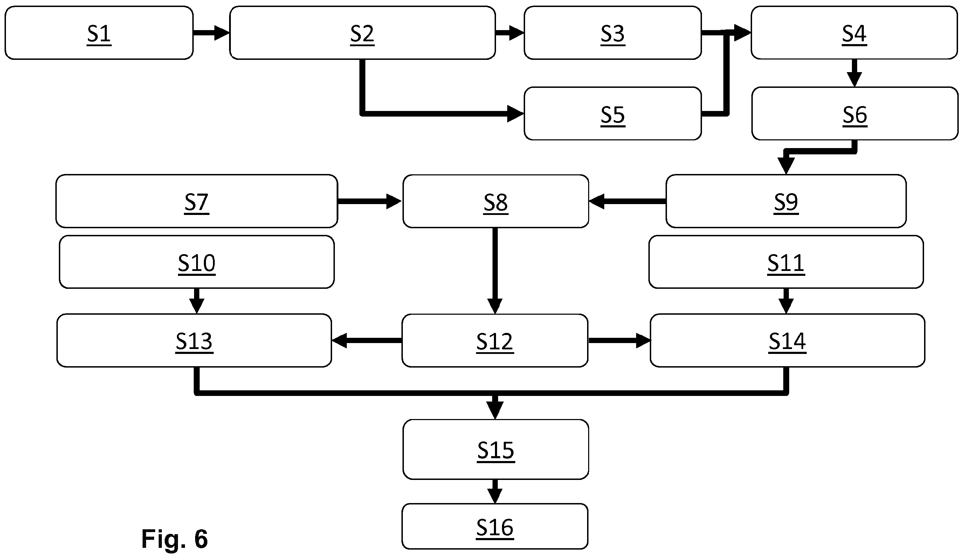

Furthermore, according to yet another embodiment shown in Fig. 6, Q-T elongation may be tracked utilizing formulae identical to Eq.s (3-1) and (3-2) by replacing the variable $Rd$ with the measured duration between the Q to the T waves (Q-T) (cf. Fig. 1).

For this, according to Fig. 6, the electrocardiogram signal S is detected by said detection element 2 (SI), and the processing unit 3 detects the Q and T complexes (QT detection algorithm, S2) in the received ECG signals S and uses time stamps for marking a Q wave and successive T wave (Q marker time stamp, S3, and T marker time stamp, S5), which are recorded (buffered outputs, S4) and used to determine the corresponding Q-T interval $QT_i$ (QT duration, S6), which is recorded (buffered QT duration outputs, S9). From these Q-T durations, a mean is determined ($\overline{QT_o}$ calculation, S8) according to

$$\text{Eq (4-1):} \qquad \overline{QT_o} = \frac{1}{n}\left(\sum_{i=1}^{n} QT_i\right)$$

wherein the number $n$ of recorded Q-T intervals can be selected/programmed (programmable sample period, S7).

Particularly, determination of the individual Q-T interval may be most easily implemented by measuring from the peak (absolute maximum for each complex) or preferably through other methods such as a start or end detection method. In the instance the Q wave is not present or has poor resolution, the P-wave or R-wave may be utilized as a non-superior alternative.

Furthermore, the determined means are recorded ($\overline{QT_o}$ stored history, S12) so as to have a history of said means and differences $\varDelta QT$ are determined ($\varDelta QT$ calculation, S15) according to

$$\text{Eq (4-2):} \qquad \varDelta QT = \overline{QT_y} - \overline{QT_x}$$

using programmed values (S13, S14), namely a more recent value $\overline{QT_y}$ and an earlier value $\overline{QT_x}$.

Particularly, the individual differences $\varDelta QT$ are recorded (stored history, S16) and may be displayed graphically or as calculated by Equation (4-2).

A value greater than zero for $\varDelta QT$ as shown in Eq (4-2) or a positive correlation between time and the equivalent values $\overline{QT_o}$ obtained from Eq (4-1) may be diagnostic of a worsening of a HF/CDV (e.g. indicator of pump failure, electrical conduction changes, ischaemia, risk for arrhythmia, rhythm disturbances, or ventricular aneurysm).

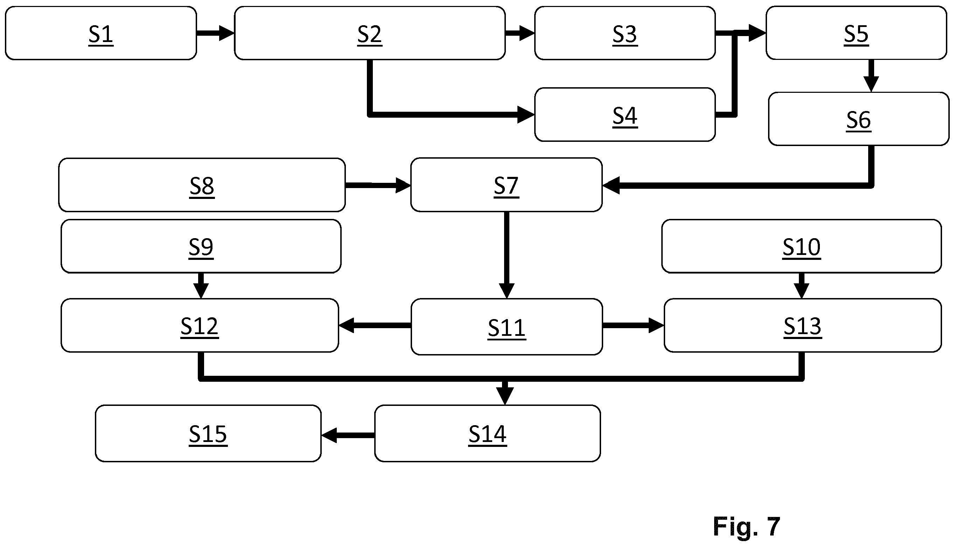

Finally, according to the embodiment shown in Fig. 7, the ratio between the absolute value of the R and S complexes, as calculated in Equation (5-1), may provide information diagnostic to RVH and LVH. In particular, devices implanted with the ECG axis oriented parallel to the midline or in a positive deviation toward the left of the midline but less than perpendicular to midline, will exhibit an increase in the R:S ratio over time in the event RVH is worsening, while the value will decrease from the time of implant if LVH is worsening. The same formulae and methodology associated with comparing values, as performed for Q-T elongation and R-R duration, may be utilized to provide this diagnostic data.

Particularly, according to Fig. 7, the electrocardiogram signal S is detected by said detection element 2 (S1), and the processing unit 3 detects the R and S complexes (RS detection algorithm, S2) in the received ECG signals S and determines their amplitudes (R amplitude, S3, and S amplitude, S4). From these amplitudes the respective $R{:}S$ ratio (($R{:}S$) calculation, S5) is determined according to

$$\text{Eq (5-1):} \qquad (R{:}S) = \frac{|R_{peak}|}{|S_{peak}|}$$

and recorded (buffered ($R{:}S$) outputs, S6). From these R:S ratios, a mean is determined ($\overline{(R{:}S)_o}$ calculation, S7) according to

$$\text{Eq (5-2):} \qquad \overline{(R{:}S)_o} = \frac{1}{n}\left(\sum_{i=1}^{n} (R{:}S)_i\right)$$

wherein the number n of recorded ratios can be selected/programmed (programmable sample period, S8).

Furthermore, the determined means $\overline{(R:S)}_o$ are recorded (stored history, S11) so as to have a history of said means and differences $\Delta(R:S)$ are determined ($\Delta(R:S)$ calculation, S14) according to

$$\text{Eq (5-3):} \qquad \Delta(R:S) = \overline{(R:S)}_y - \overline{(R:S)}_x$$

using programmed values (S12, S13), namely a more recent value $\overline{(R:S)}_y$ and an earlier value $\overline{(R:S)}_x$.

Particularly, the individual differences $\Delta(R:S)$ are recorded (stored history, S15) and may be displayed graphically or as calculated by Equation (5-3).

Further, particularly, a value greater than zero for the difference $\Delta(R:S)$ or a positive correlation between time and the means $\overline{(R:S)}_0$ points to a worsening of RVH, and a value smaller than zero for the difference $\Delta(R:S)$ or a negative correlation between time and the means $\overline{(R:S)}_0$ points to a worsening of LVH.

In Fig. 5 and 7, S9 and S10 refer to a programmable sample period, respectively. In Fig. 6, S10 and S11 refer to a programmable sample period, respectively.

## Claims

1. An implantable device (1) for monitoring of a patient's electrocardiogram (ECG), the device (1) comprising a detection element (2) for determining electrocardiogram signals (S) of a heart (H) of the patient, which signals (S) are indicative of the electrocardiogram (ECG) of the patient, wherein the implantable device (1) comprises a processing unit (3) that is configured to determine from said signals (S) at least one parameter of the electrocardiogram (ECG) or a quantity derived therefrom.

2. The device according to claim 1, **wherein** the processing unit (3) is configured to record a plurality of QRS amplitudes ($QRS_{(i-t)}$) during a first sampling period and a plurality of QRS amplitudes ($QRS_{(i)}$) during a later second sampling period, and to determine a net change of the QRS amplitude ($\overline{\Delta QRS}$) between the two sampling periods using the relation $\overline{\Delta QRS} = \overline{QRS}_{(i)} - \overline{QRS}_{(i-t)}$, wherein $\overline{QRS}_{(i)}$ is the average QRS amplitude of the second sampling period and wherein $\overline{QRS}_{(i-t)}$ is the average QRS amplitude of the earlier first sampling period.

3. The device according to claim 2, **wherein** the processing unit (3) is configured to determine and record a plurality of net changes of the QRS amplitude ($\overline{\Delta QRS}$) as a function of time, particularly from the time at which the implantable device (1) has been implanted and started for the first time until the time at which the net change of the QRS amplitude ($\overline{\Delta QRS}$) has been determined most recently.

4. The device according to one of the preceding claims, **wherein** the processing unit (3) is configured to determine and record a plurality of R-R durations ($Rd_i$).

5. The device according to claim 4, **wherein** the processing unit (3) is configured to determine a measure ($\overline{Rd}$), particularly a mean, of said plurality of R-R durations, wherein the processing unit (3) is further configured to determine a variance $S^2_{(Rd)}$ of said measure ($\overline{Rd}$) using the relation $S^2_{(Rd)} = \frac{1}{n-1}\sum_{i=1}^{n}(Rd_i - \overline{Rd})^2$, wherein $n$ is the number of recorded R-R durations, $Rd_i$ is the i-th recorded R-R duration, and $\overline{Rd}$ is said measure.

6. The device according to claim 5, **wherein** the processing unit (3) is configured to determine and record a plurality of variances $(S^2_{(Rd)})$ of measures ($\overline{Rd}$) of R-R durations as a function of time.

7. The device according to claim 6, **wherein** the processing unit (3) is configured to determine and record at least one or a plurality of F values according to $F = \frac{S^2_{Rd(\alpha)}}{S^2_{Rd(\beta)}}$, wherein $S^2_{Rd(\alpha)}$ is a variance of said plurality of variances that has been determined more recently than the variance $S^2_{Rd(\beta)}$, which is a variance of said plurality of variances, too.

8. The device according to claim 7, **wherein** the processing unit (3) is configured to determine and record a difference $\Delta F$ between two of the recorded F values $Fx$ and $Fy$ according to $\Delta F = Fy - Fx$, wherein $Fx$ has been determined and recorded before $Fy$, and wherein particularly the processing unit (3) is configured to determine and record a plurality of differences of recorded F values.

9. The device according to one of the preceding claims, **wherein** the processing unit (3) is configured to determine and record a plurality of Q-T durations ($QT_i$).

10. Device according to claim 9, **wherein** the processing unit (3) is configured to determine a mean ($\overline{QT}_0$) of said plurality of Q-T durations according to $\overline{QT}_o = \dfrac{1}{n}\left(\sum_{i=1}^{n} QT_i\right),$ wherein n is the number of recorded Q-T durations, and $QT_i$ is the i-th recorded Q-T duration.

11. The device according to claim 10, **wherein** the processing unit (3) is configured to determine and record a plurality of means ($\overline{QT}_0$) of Q-T durations as a function of time.

12. The device according to claim 11, **wherein** the processing unit (3) is configured to determine and record a difference $\Delta QT$ between two of the recorded means $\overline{QT}_x$ and $\overline{QT}_y$ according to $\Delta QT = \overline{QT}_y - \overline{QT}_x$, wherein $\overline{QT}_x$ has been determined and recorded before $\overline{QT}_y$, wherein particularly the processing unit (3) is configured to determine and record a plurality of differences $\Delta QT$ between recorded means.

13. The device according to one of the preceding claims, **wherein** said at least one parameter is an R:S ratio ($R{:}S$) defined as the ratio between the absolute value ($|R_{peak}|$) of the R complex and the absolute value ($|S_{peak}|$) of the S complex.

14. The device according claim 13, **wherein** the processing unit (3) is configured to determine and record a plurality of R:S ratios (R:S).

15. Device according to claim 14, **wherein** the processing unit (3) is configured to determine a mean ($\overline{(R{:}S)}_0$) of said plurality of R:S ratios according to $\overline{(R{:}S)}_0 = \dfrac{1}{n}\left(\sum_{i=1}^{n}(R{:}S)_i\right),$ wherein n is the number of recorded R:S ratios, and $(R{:}S)_i$ is the i-th recorded R:S ratio.

Fig. 1

Fig. 2

```
┌──────────────┐        ┌────────────────────────────┐        ┌──────────────┐
│      S1      │──────▶│             S2             │──────▶│      S3      │
└──────────────┘        └────────────────────────────┘        └──────────────┘
                                                                      │
                                                                      ▼
┌──────────────────────┐   ┌──────────────┐   ┌──────────────────┐   ┌──────────────┐
│          S7          │──▶│      S6      │◀──│        S5        │◀──│      S4      │
└──────────────────────┘   └──────────────┘   └──────────────────┘   └──────────────┘
                                   │                    │
              ┌────────────────────┘                    └────────────────────┐
              ▼                                                               ▼
┌──────────────────────┐        ┌──────────────┐                     ┌──────────────┐
│          S9          │───────▶│     S10      │                     │      S8      │
└──────────────────────┘        └──────────────┘                     └──────────────┘
                                       │                                    │
                                       ▼                                    │
                             ┌──────────────────┐                           │
                             │       S11        │◀──────────────────────────┘
                             └──────────────────┘
                                       │
        ┌──────────────┐               │
        │     S12      │               │
        └──────────────┘               │
              │                        │
              └────────────────────────┘
              ▼
        ┌──────────────┐
        │     S13      │
        └──────────────┘
```

Fig. 3

Fig. 4

S1 → S2 → S3 → S4 → S5

S6 → S8 → S11 → S13

S7 → S8

S9 → S12

S10 → S13

S11 → S12

S11 → S14 → S15

S12 → S14

**Fig. 5**

Fig. 6

```
┌──────────┐    ┌──────────────┐    ┌──────────┐    ┌──────────┐
│    S1    │──▶ │      S2      │──▶ │    S3    │──▶ │    S5    │
└──────────┘    └──────────────┘    └──────────┘    └──────────┘
                       │                   │              │
                       ▼                   │              ▼
                ┌──────────────┐           │       ┌──────────┐
                │      S4      │───────────┘       │    S6    │
                └──────────────┘                   └──────────┘
                                                         │
┌──────────────┐    ┌──────────────┐                     │
│      S8      │──▶ │      S7      │ ◀───────────────────┘
└──────────────┘    └──────────────┘
┌──────────────┐           │              ┌──────────────┐
│      S9      │           ▼              │     S10      │
└──────────────┘    ┌──────────────┐      └──────────────┘
       │            │     S11      │             │
       ▼            └──────────────┘             ▼
┌──────────────┐      │        │          ┌──────────────┐
│     S12      │ ◀────┘        └────────▶ │     S13      │
└──────────────┘                          └──────────────┘
       │                                         │
       └──────────────┐       ┌─────────────────┘
                      ▼       ▼
            ┌──────────────┐     ┌──────────────┐
            │     S15      │ ◀── │     S14      │
            └──────────────┘     └──────────────┘
```

**Fig. 7**

Europäisches
Patentamt

European
Patent Office

Office européen
des brevets

# EUROPEAN SEARCH REPORT

Application Number

EP 18 15 8139

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 294 972 A1 (PACESETTER INC [US]) 16 March 2011 (2011-03-16) <br> * page 5, lines 31, 33, 34 * <br> * page 6, lines 28, 29 * <br> * page 7, lines 56, 57 * <br> * page 9, lines 22, 24, 38, 40 * <br> * page 10, lines 31-33 * <br> * page 12, lines 36, 40 * | 1,4-15 | INV. <br> A61B5/0472 <br> A61B5/0456 <br> A61B5/024 <br> A61B5/02 <br> A61B5/00 |
| X | US 2004/215254 A1 (BOUTE WILLEM [NL] ET AL) 28 October 2004 (2004-10-28) <br> * paragraphs [0055], [0058], [0059] * <br> * figures 1-5 * | 1-3, 13-15 | |
| X | US 2009/099426 A1 (SACHANANDANI HARESH G [US] ET AL) 16 April 2009 (2009-04-16) <br> * figures 1, 3, 4 * | 1 | |
| A | US 2010/030293 A1 (SARKAR SHANTANU [US] ET AL) 4 February 2010 (2010-02-04) <br> * paragraphs [0157], [0159] * | 7,8 | |
| A | John Madias: "The Resting Eelctrocardiogram in the Management of Patients with Congestive Heart Failure", PACE, 1 January 2007 (2007-01-01), pages 123-128, XP055510097, Retrieved from the Internet: URL:https://onlinelibrary.wiley.com/doi/ep df/10.1111/j.1540-8159.2007.00586.x [retrieved on 2018-09-26] <br> * page 124, right-hand column, lines 13-15 * | 13-15 | TECHNICAL FIELDS SEARCHED (IPC) <br><br> A61B |
| A | US 2015/141859 A1 (SANDLER ALEX [US] ET AL) 21 May 2015 (2015-05-21) <br> * the whole document * | 1-15 | |

-/--

The present search report has been drawn up for all claims

| Place of search | Date of completion of the search | Examiner |
|---|---|---|
| The Hague | 26 September 2018 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 15 8139

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/257122 A1 (ONG MARCUS ENG HOCK [SG] ET AL) 11 September 2014 (2014-09-11) * the whole document * | 1-15 | |

TECHNICAL FIELDS
SEARCHED      (IPC)

The present search report has been drawn up for all claims

| Place of search | Date of completion of the search | Examiner |
|---|---|---|
| The Hague | 26 September 2018 | Meyer, Wolfgang |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

&  : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 18 15 8139

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2018

| Patent document cited in search report | | Publication date | Patent family member(s) | | Publication date |
|---|---|---|---|---|---|
| EP 2294972 | A1 | 16-03-2011 | EP 2294972 A1 | | 16-03-2011 |
| | | | US 2011066055 A1 | | 17-03-2011 |
| US 2004215254 | A1 | 28-10-2004 | EP 1617902 A2 | | 25-01-2006 |
| | | | US 2004215254 A1 | | 28-10-2004 |
| | | | WO 2004096035 A2 | | 11-11-2004 |
| US 2009099426 | A1 | 16-04-2009 | EP 2219518 A1 | | 25-08-2010 |
| | | | JP 5119334 B2 | | 16-01-2013 |
| | | | JP 2011500145 A | | 06-01-2011 |
| | | | US 2009099426 A1 | | 16-04-2009 |
| | | | US 2013211266 A1 | | 15-08-2013 |
| | | | US 2014107503 A1 | | 17-04-2014 |
| | | | US 2015245774 A1 | | 03-09-2015 |
| | | | US 2016242655 A1 | | 25-08-2016 |
| | | | US 2017100080 A1 | | 13-04-2017 |
| | | | WO 2009051658 A1 | | 23-04-2009 |
| US 2010030293 | A1 | 04-02-2010 | US 2010030293 A1 | | 04-02-2010 |
| | | | US 2015230722 A1 | | 20-08-2015 |
| | | | US 2017360320 A1 | | 21-12-2017 |
| US 2015141859 | A1 | 21-05-2015 | NONE | | |
| US 2014257122 | A1 | 11-09-2014 | CN 105228508 A | | 06-01-2016 |
| | | | EP 2964077 A1 | | 13-01-2016 |
| | | | SG 11201507172X A | | 29-10-2015 |
| | | | US 2014257122 A1 | | 11-09-2014 |
| | | | US 2016022162 A1 | | 28-01-2016 |
| | | | WO 2014137295 A1 | | 12-09-2014 |

EPO FORM P0459

For more details about this annex : see Official Journal of the European Patent Office, No. 12/82

**EP 3 508 117 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040116819 A1